# EUROPÄISCHE PATENTSCHRIFT

(11) **EP 1 206 555 B1**
(45) Veröffentlichungstag und Bekanntmachung des Hinweises auf die Patenterteilung: **19.11.2008**
(21) Anmeldenummer: 00958214.9
(22) Anmeldetag: 02.08.2000
(51) Int. Cl.: C12N 15/70, C07K 19/00, C12N 1/21, A61K 39/395, A61P 35/00

(54) **FV-ANTIKÖRPER-KONSTRUKTE MIT BINDUNGSSTELLEN FÜR EINEN CD16-REZEPTOR UND EIN CD30-OBERFLÄCHENPROTEIN**
FV ANTIBODY CONSTRUCT COMPRISING BINDING SITES FOR A CD16 RECEPTOR AND A CD30 SURFACE PROTEIN
CONSTRUCTION D'ANTICORPS FV COMPORTANT DES SITES DE LIAISON POUR UN RECEPTEUR CD16 ET UNE PROTEINE DE SURFACE CD30

(30) Priorität: 06.08.1999 DE 19937264
(43) Veröffentlichungstag der Anmeldung: 22.05.2002
(73) Patentinhaber: Deutsches Krebsforschungszentrum Stiftung des öffentlichen Rechts, 69120 Heidelberg (DE)
(72) Erfinder: ARNDT, Michaela, D-66459 Kirkel-Limbach (DE); LITTLE, Melvyn, D-69151 Neckargemünd (DE); KYPRIYANOV, Sergey, D-69121 Heidelberg (DE); KRAUSS, Jürgen, D-66459 Kirkel-Limbach (DE); PFREUNDSCHUH, Michael, D-66424 Homburg/Saar (DE)
(74) Vertreter: Schüssler, Andrea
(86) Internationale Anmeldenummer: PCT/DE2000/002589
(87) Internationale Veröffentlichungsnummer: WO 2001/011059

(56) Entgegenhaltungen:
- DE-A- 19 531 346
- DE-A- 19 838 967
- DE-C- 4 337 197
- S. KIPRIYANOV ET AL.: "Bispecific CD3xCD19 diabody for T cell-mediated lysis of malignant human B cells." INTERNATIONAL JOURNAL OF CANCER, Bd. 77, Nr. 5, 31. August 1998 (1998-08-31), Seiten 763-772, XP002115487 Basel, die Schweiz
- A. KLIMKA ET AL.: "Making anti-CD30 scFv recombinant antibodies from their hybridomas using phage display technology." ANNALS OF HEMATOLOGY, Bd. 73, Nr. suppl. 2, 1996, Seite A153 XP000653169 Berlin, Deutschland
- M. ARNDT ET AL.: "A bispecific diabody that mediates natural killer cell cytotoxicity against xenotransplantated human Hodgkin's tumors." BLOOD, Bd. 94, Nr. 8, 15. Oktober 1999 (1999-10-15), Seiten 2562-2568, XP002153101 New York, NY, VSA

### SEQUENZPROTOKOLL

<110> Deutsches Krebsforschungszentrum
<120> Fv-Antikörper-Konstrukte
<130> K 2839
<140> unbekannt
<150> DE 199 37 264.0
   <151> 1999-08-06
<160> 11
<170> PatentIn Ver. 2.1
<210> 1
   <211> 4570
   <212> DNA
   <213> Künstliche Sequenz
<220>
   <223> Beschreibung der künstlichen Sequenz: Expressionsvektor kodierend für zwei einzelkettige Fv-Antikörper-Konstrukte
<220>
   <221> CDS
   <222> (217)..(1002)
<220>
   <221> CDS
   <222> (1102)..(1920)
<400> 1
   ctcgagagcg ggcagtgagc gcaacgcaat taatgtgagt tagctcactc attaggcacc 60
   ccaggcttta cactttatgc tcccggctcg tatgttgtgt ggaattgtga gcggataaca 120
   atttcacaca gaattcatta aagaggagaa attaaccatg aaatacctat tgcctacggc 180
   agccgctggc ttgctgctgc tggcagctca gccgcc atg gcg cag gtg cag ctg  234
                                           Met Ala Gln Val Gln Leu
                                           1               5
   cag cag tct gga gct gag ctg gta agg cct ggg act tca gtg aag ata  282
   Gln Gln Ser Gly Ala Glu Leu Val Arg Pro Gly Thr Ser Val Lys Ile
               10                  15                  20
   tcc tgc aag gct tct ggc tac acc ttc act aac tac tgg cta ggt tgg  330
   Ser Cys Lys Ala Ser Gly Tyr Thr Phe Thr Asn Tyr Trp Leu Gly Trp
           25                  30                  35
   gta aaa cag agg cct gga cat gga ctc gag tgg att gga gat atc tac  378
   Val Lys Gln Arg Pro Gly His Gly Leu Glu Trp Ile Gly Asp Ile Tyr
       40                  45                  50
   cct gga ggt ggt tat act aac tac aat gag aaa ttc aag ggc aag gcc  426
   Pro Gly Gly Gly Tyr Thr Asn Tyr Asn Glu Lys Phe Lys Gly Lys Ala
   55                  60                  65                  70
   aca gtg act gca gac aca tcc tcc aga act gcc tac gtg cag gtc agg    474
   Thr Val Thr Ala Asp Thr Ser Ser Arg Thr Ala Tyr Val Gln Val Arg
                   75                  80                  85
   agc ctg aca tct gag gac tct gct gtc tat ttc tgt gca aga tcg gct    522
   Ser Leu Thr Ser Glu Asp Ser Ala Val Tyr Phe Cys Ala Arg Ser Ala
               90                  95                  100
   agc tgg tac ttc gat gtc tgg ggc gca cgg act acg gtc acc gtc tcc    570
   Ser Trp Tyr Phe Asp Val Trp Gly Ala Arg Thr Thr Val Thr Val Ser
           105                 110                 115
   tca gcc aaa aca aca ccc aag ctt ggc ggt gat atc gag ctc act cag    618
   Ser Ala Lys Thr Thr Pro Lys Leu Gly Gly Asp Ile Glu Leu Thr Gln
       120                 125                 130
   tct cca aaa ttc atg tcc aca tca gta gga gac agg gtc aac gtc acc    666
   Ser Pro Lys Phe Met Ser Thr Ser Val Gly Asp Arg Val Asn Val Thr
   135                 140                 145                 150
   tac aag gcc agt cag aat gtg ggt act aat gta gcc tgg ttt caa caa    714
   Tyr Lys Ala Ser Gln Asn Val Gly Thr Asn Val Ala Trp Phe Gln Gln
                   155                 160                 165
   aaa cca ggg caa tct cct aaa gtt ctg att tac tcg gca tct tac cga    762
   Lys Pro Gly Gln Ser Pro Lys Val Leu Ile Tyr Ser Ala Ser Tyr Arg
               170                 175                 180
   tac agt gga gtc cct gat cgc ttc aca ggc agt gga tct gga aca gat    810
   Tyr Ser Gly Val Pro Asp Arg Phe Thr Gly Ser Gly Ser Gly Thr Asp
           185                 190                 195
   ttc act ctc acc atc agc aat gtg cag tct gaa gac ttg gca gag tat    858
   Phe Thr Leu Thr Ile Ser Asn Val Gln Ser Glu Asp Leu Ala Glu Tyr
       200                 205                 210
   ttc tgt cag caa tat cac acc tat cct ctc acg ttc gga ggg ggc acc    906
   Phe Cys Gln Gln Tyr His Thr Tyr Pro Leu Thr Phe Gly Gly Gly Thr
   215                 220                 225                 230
   aag ctg gaa atc aaa cgg gct gat gct gcg gcc gct gga tcc gaa caa    954
   Lys Leu Glu Ile Lys Arg Ala Asp Ala Ala Ala Ala Gly Ser Glu Gln
                   235                 240                 245
   aag ctg atc tca gaa gaa gac cta aac tca cat cac cat cac cat cac    1002
   Lys Leu Ile Ser Glu Glu Asp Leu Asn Ser His His His His His His
               250                 255                 260
   taaagatcta ttaaagagga gaaattaacc atgaaatacc tattgcctac ggcagccgct 1062
   ggcttgctgc tgctggcagc tcagccggcc atggcggcc atg gcc cag gtg caa    1116
                                             Met Ala Gln Val Gln
                                                         265
   ctg cag cag tca ggg gct gag ctg gct aga cct ggg gct tca gtg aag    1164
   Leu Gln Gln Ser Gly Ala Glu Leu Ala Arg Pro Gly Ala Ser Val Lys
           270                 275                 280
   atg tcc tgc aag gct tct ggc tac acc ttt act acc tac aca ata cac    1212
   Met Ser Cys Lys Ala Ser Gly Tyr Thr Phe Thr Thr Tyr Thr Ile His
       285                 290                 295
   tgg gta aga cag agg cct gga cac gat ctg gaa tgg att gga tac att    1260
   Trp Val Arg Gln Arg Pro Gly His Asp Leu Glu Trp Ile Gly Tyr Ile
   300                 305                 310                 315
   aat cct agc agt gga tat tct gac tac aat cag aac ttc aag ggc aag     1308
   Asn Pro Ser Ser Gly Tyr Ser Asp Tyr Asn Gln Asn Phe Lys Gly Lys
                   320                 325                 330
   acc aca ttg act gca gac aag tcc tcc aac aca gcc tac atg caa ctg     1356
   Thr Thr Leu Thr Ala Asp Lys Ser Ser Asn Thr Ala Tyr Met Gln Leu
               335                 340                 345
   aac agc ctg aca tct gag gac tct gcg gtc tat tac tgt gca aga aga     1404
   Asn Ser Leu Thr Ser Glu Asp Ser Ala Val Tyr Tyr Cys Ala Arg Arg
           350                 355                 360
   gcg gac tat ggt aac tac gaa tat acc tgg ttt gct tac tgg ggc caa     1452
   Ala Asp Tyr Gly Asn Tyr Glu Tyr Thr Trp Phe Ala Tyr Trp Gly Gln
       365                 370                 375
   ggg acc acg gtc acc gtc tcc tca gcc aaa aca aca ccc aag ctt ggc     1500
   Gly Thr Thr Val Thr Val Ser Ser Ala Lys Thr Thr Pro Lys Leu Gly
   380                 385                 390                 395
   ggt gat atc cag gct gtt gtg act cag gaa tct gca ctc acc aca tca     1548
   Gly Asp Ile Gln Ala Val Val Thr Gln Glu Ser Ala Leu Thr Thr Ser
                   400                 405                 410
   cct ggt gaa aca gtc aca ctc act tgt cgc tca aat act ggg act gtt     1596
   Pro Gly Glu Thr Val Thr Leu Thr Cys Arg Ser Asn Thr Gly Thr Val
               415                 420                 425
   aca act agt aac tat gcc aac tgg gtc caa gaa aaa cca gat cat tta     1644
   Thr Thr Ser Asn Tyr Ala Asn Trp Val Gln Glu Lys Pro Asp His Leu
           430                 435                 440
   ttc act ggt cta ata ggt cat acc aac aac cga gct cca ggt gtt cct     1692
   Phe Thr Gly Leu Ile Gly His Thr Asn Asn Arg Ala Pro Gly Val Pro
       445                 450                 455
   gcc aga ttc tca ggc tcc ctg att gga gac aag gct gcc ctc acc atc     1740
   Ala Arg Phe Ser Gly Ser Leu Ile Gly Asp Lys Ala Ala Leu Thr Ile
   460                 465                 470                 475
   aca ggg gca cag act gag gat gag gca ata tat ttc tgt gct cta tgg     1788
   Thr Gly Ala Gln Thr Glu Asp Glu Ala Ile Tyr Phe Cys Ala Leu Trp
                   480                 485                 490
   tat aac aac cat tgg gtg ttc ggt gga gga acc aaa ctg act gtc cta     1836
   Tyr Asn Asn His Trp Val Phe Gly Gly Gly Thr Lys Leu Thr Val Leu
               495                 500                 505
   ggc cag ccc aag tct gcg gcc gct gga tcc gaa caa aag ctg atc tca     1884
   Gly Gln Pro Lys Ser Ala Ala Ala Gly Ser Glu Gln Lys Leu Ile Ser
           510                 515                 520
   gaa gaa gac cta aac tca cat cac cat cac cat cac taatctagag          1930
   Glu Glu Asp Leu Asn Ser His His His His His His
       525                 530                 535
   gcctgtgcta atgatcagct agcttgaggc atcaataaaa cgaaaggctc agtcgaaaga 1990
   ctgggccttt cgttttatct gttgtttgtc ggttaacgtc gacctggcgt aatagcgaga 2050
   ggcccgcacc gatcgccctt cccaacagtt gcgcagcctg aatggcgaat gggacgcgcc 2110
   ctgtagcggc gcattaagcg cggcgggtgt ggtggttacg cgcagcgtga ccgctacact 2170
   tgccagcgcc ctagcgcccg ctcctttcgc tttcttccct tcctttctcg ccacgttcgc 2230
   cggctttccc cgtcaagctc taaatcgggg gctcccttta gggttccgat ttagtgcttt 2290
   acggcacctc gaccccaaaa aacttgatta gggtgatggt tcacgtagtg ggccatcgcc 2350
   ctgatagacg gtttttcgcc ctttgacgtt ggagtccacg ttctttaata gtggactctt 2410
   gttccaaact ggaacaacac tcaaccctat ctcggtctat tcttttgatt tataagggat 2470
   tttgccgatt tcggcctatt ggttaaaaaa tgagctgatt taacaaaaat ttaacgcgaa 2530
   ttttaacaaa atattaacgc ttacaattta ggtggcactt ttcggggaaa tgtgcgcgga 2590
   acccctattt gtttattttt ctaaatacat tcaaatatgt atccgctcat gagacaataa 2650
   ccctgataaa tgcttcaata atattgaaaa aggaagagta tgagtattca acatttccgt 2710
   gtcgccctta ttcccttttt tgcggcattt tgccttcctg tttttgctca cccagaaacg 2770
   ctggtgaaag taaaagatgc tgaagatcag ttgggtgcac gagtgggtta catcgaactg 2830
   gatctcaaca gcggtaagat ccttgagagt tttcgccccg aagaacgttt tccaatgatg 2890
   agcactttta aagttctgct atgtggcgcg gtattatccc gtattgacgc cgggcaagag 2950
   caactcggtc gccgcataca ctattctcag aatgacttgg ttgagtactc accagtcaca 3010
   gaaaagcatc ttacggatgg catgacagta agagaattat gcagtgctgc cataaccatg 3070
   agtgataaca ctgcggccaa cttacttctg acaacgatcg gaggaccgaa ggagctaacc 3130
   gcttttttgc acaacatggg ggatcatgta actcgccttg atcgttggga accggagctg 3190
   aatgaagcca taccaaacga cgagcgtgac accacgatgc ctgtagcaat ggcaacaacg 3250
   ttgcgcaaac tattaactgg cgaactactt actctagctt cccggcaaca attaatagac 3310
   tggatggagg cggataaagt tgcaggacca cttctgcgct cggcccttcc ggctggctgg 3370
   tttattgctg ataaatctgg agccggtgag cgtgggtctc gcggtatcat tgcagcactg 3430
   gggccagatg gtaagccctc ccgtatcgta gttatctaca cgacggggag tcaggcaact 3490
   atggatgaac gaaatagaca gatcgctgag ataggtgcct cactgattaa gcattggtaa 3550
   ctgtcagacc aagtttactc atatatactt tagattgatt taaaacttca tttttaattt 3610
   aaaaggatct aggtgaagat cctttttgat aatctcatga ccaaaatccc ttaacgtgag 3670
   ttttcgttcc actgagcgtc agaccccgta gaaaagatca aaggatcttc ttgagatcct 3730
   ttttttctgc gcgtaatctg ctgcttgcaa acaaaaaaac caccgctacc agcggtggtt 3790
   tgtttgccgg atcaagagct accaactctt tttccgaagg taactggctt cagcagagcg 3850
   cagataccaa atactgtcct tctagtgtag ccgtagttag gccaccactt caagaactct 3910
   gtagcaccgc ctacatacct cgctctgcta atcctgttac cagtggctgc tgccagtggc 3970
   gataagtcgt gtcttaccgg gttggactca agacgatagt taccggataa ggcgcagcgg 4030
   tcgggctgaa cggggggttc gtgcacacag cccagcttgg agcgaacgac ctacaccgaa 4090
   ctgagaacct acagcgtgag ctatgagaaa gcgccacgct tcccgaaggg agaaaggcgg 4150
   acaggtatcc ggtaagcggc agggtcggaa caggagagcg cacgagggag cttccagggg 4210
   gaaacgcctg gtatctttat agtcctgtcg ggtttcgcca cctctgactt gagcgtcgat 4270
   ttttgtgatg ctcgtcaggg gggcggagcc tatggaaaaa cgccagcaac gcggcctttt 4330
   tacggttcct ggccttttgc tggccttttg ctcacatgtt ctttcctgcg ttatcccctg 4390
   attctgtgga taaccgtatt accgcctttg agtgagctga taccgctcgc cgcagccgaa 4450
   cgaccgagcg cagcgagtca gtgagcgagg aagcggaaga gcgcccaata cgcaaaccgc 4510
   ctctccccgc gcgttggccg attcattaat gcaggtatca cgaggccctt tcgtcttcac 4570
<210> 2
   <211> 262
   <212> PRT
   <213> Künstliche Sequenz
   <223> Beschreibung der künstlichen Sequenz: Expressionsvektor kodierend für zwei einzelkettige Fv-Antikörper-Konstrukte
<400> 2
   Met Ala Gln Val Gln Leu Gln Gln Ser Gly Ala Glu Leu Val Arg Pro
   1               5                   10                  15
   Gly Thr Ser Val Lys Ile Ser Cys Lys Ala Ser Gly Tyr Thr Phe Thr
               20                  25                  30
   Asn Tyr Trp Leu Gly Trp Val Lys Gln Arg Pro Gly His Gly Leu Glu
           35                  40                  45
   Trp Ile Gly Asp Ile Tyr Pro Gly Gly Gly Tyr Thr Asn Tyr Asn Glu
       50                  55                  60
   Lys Phe Lys Gly Lys Ala Thr Val Thr Ala Asp Thr Ser Ser Arg Thr
   65                  70                  75                  80
   Ala Tyr Val Gln Val Arg Ser Leu Thr Ser Glu Asp Ser Ala Val Tyr
                   85                  90                  95
   Phe Cys Ala Arg Ser Ala Ser Trp Tyr Phe Asp Val Trp Gly Ala Arg
               100                 105                 110
   Thr Thr Val Thr Val Ser Ser Ala Lys Thr Thr Pro Lys Leu Gly Gly
           115                 120                 125
   Asp Ile Glu Leu Thr Gln Ser Pro Lys Phe Met Ser Thr Ser Val Gly
       130                 135                 140
   Asp Arg Val Asn Val Thr Tyr Lys Ala Ser Gln Asn Val Gly Thr Asn
   145                 150                 155                 160
   Val Ala Trp Phe Gln Gln Lys Pro Gly Gln Ser Pro Lys Val Leu Ile
                   165                 170                 175
   Tyr Ser Ala Ser Tyr Arg Tyr Ser Gly Val Pro Asp Arg Phe Thr Gly
               180                 185                 190
   Ser Gly Ser Gly Thr Asp Phe Thr Leu Thr Ile Ser Asn Val Gln Ser
           195                 200                 205
   Glu Asp Leu Ala Glu Tyr Phe Cys Gln Gln Tyr His Thr Tyr Pro Leu
       210                 215                 220
   Thr Phe Gly Gly Gly Thr Lys Leu Glu Ile Lys Arg Ala Asp Ala Ala
   225                 230                 235                 240
   Ala Ala Gly Ser Glu Gln Lys Leu Ile Ser Glu Glu Asp Leu Asn Ser
                   245                 250                 255
   His His His His His His
               260
<210> 3
   <211> 273
   <212> PRT
   <213> Künstliche Sequenz
   <223> Beschreibung der künstlichen Sequenz: Expressionsvektor kodierend für zwei einzelkettige Fv-Antikörper-Konstrukte
<400> 3
   Met Ala Gln Val Gln Leu Gln Gln Ser Gly Ala Glu Leu Ala Arg Pro
   1               5                   10                  15
   Gly Ala Ser Val Lys Met Ser Cys Lys Ala Ser Gly Tyr Thr Phe Thr
               20                  25                  30
   Thr Tyr Thr Ile His Trp Val Arg Gln Arg Pro Gly His Asp Leu Glu
           35                  40                  45
   Trp Ile Gly Tyr Ile Asn Pro Ser Ser Gly Tyr Ser Asp Tyr Asn Gln
       50                  55                  60
   Asn Phe Lys Gly Lys Thr Thr Leu Thr Ala Asp Lys Ser Ser Asn Thr
   65                  70                  75                  80
   Ala Tyr Met Gln Leu Asn Ser Leu Thr Ser Glu Asp Ser Ala Val Tyr
                   85                  90                  95
   Tyr Cys Ala Arg Arg Ala Asp Tyr Gly Asn Tyr Glu Tyr Thr Trp Phe
               100                 105                 110
   Ala Tyr Trp Gly Gln Gly Thr Thr Val Thr Val Ser Ser Ala Lys Thr
           115                 120                 125
   Thr Pro Lys Leu Gly Gly Asp Ile Gln Ala Val Val Thr Gln Glu Ser
       130                 135                 140
   Ala Leu Thr Thr Ser Pro Gly Glu Thr Val Thr Leu Thr Cys Arg Ser
   145                 150                 155                 160
   Asn Thr Gly Thr Val Thr Thr Ser Asn Tyr Ala Asn Trp Val Gln Glu
                   165                 170                 175
   Lys Pro Asp His Leu Phe Thr Gly Leu Ile Gly His Thr Asn Asn Arg
               180                 185                 190
   Ala Pro Gly Val Pro Ala Arg Phe Ser Gly Ser Leu Ile Gly Asp Lys
           195                 200                 205
   Ala Ala Leu Thr Ile Thr Gly Ala Gln Thr Glu Asp Glu Ala Ile Tyr
       210                 215                 220
   Phe Cys Ala Leu Trp Tyr Asn Asn His Trp Val Phe Gly Gly Gly Thr
   225                 230                 235                 240
   Lys Leu Thr Val Leu Gly Gln Pro Lys Ser Ala Ala Ala Gly Ser Glu
                   245                 250                 255
   Gln Lys Leu Ile Ser Glu Glu Asp Leu Asn Ser His His His His His
               260                 265                 270
   His
<210> 4
   <211> 35
   <212> DNA
   <213> Künstliche Sequenz
<220>
   <223> Beschreibung der künstlichen Sequenz: Primer
<400> 4
   cagccggcca tggcgcaggt cgcagctgca gcgag 35
<210> 5
   <211> 38
   <212> DNA
   <213> Künstliche Sequenz
<220>
   <223> Beschreibung der künstlichen Sequenz: Primer
<400> 5
   ccaggggcca gtggatagac aagcttgggt gttgtttt 38
<210> 6
   <211> 30
   <212> DNA
   <213> Künstliche Sequenz
<220>
   <223> Beschreibung der künstlichen Sequenz: Primer
<400> 6
   agagacgcgt acaggctgtt gtgactcagg 30
<210> 7
   <211> 27
   <212> DNA
   <213> Künstliche Sequenz
<220>
   <223> Beschreibung der künstlichen Sequenz: Primer
<400> 7
   gactgcggcc gcagacttgg gctggcc 27
<210> 8
   <211> 21
   <212> DNA
   <213> Künstliche Sequenz
<220>
   <223> Beschreibung der künstlichen Sequenz: Primer
<400> 8
   atgaccatga ttacgccaag c 21
<210> 9
   <211> 36
   <212> DNA
   <213> Künstliche Sequenz
<220>
   <223> Beschreibung der künstlichen Sequenz: Primer
<400> 9
   agacaagctt gggtgttgtt ttggctgagg agacgg 36
<210> 10
   <211> 27
   <212> DNA
   <213> Künstliche Sequenz
<220>
<223> Beschreibung der künstlichen Sequenz: Primer
<400> 10
   ggcggatatc gagctcactc agtctcc 27
<210> 11
   <211> 34
   <212> DNA
   <213> Künstliche Sequenz
<220>
   <223> Beschreibung der künstlichen Sequenz: Primer
<400> 11
   tatagcggcc gcagcatcag cccgtttgat ttcc 34

## Beschreibung

Die vorliegende Erfindung betriff Fᵥ-Antikörper-Konstrukte, die eine Regression von Morbus - Hodgkin induzieren können, für solche Fᵥ-Antikörper-Konstrukte kodierende DNAs sowie ein Verfahren zur Herstellung der Fᵥ-Antikörper-Konsbukte und ihre Verwendung.

Natürliche Antikörper weisen vier variable Domänen, zwei V_{H}- und zwei V_{L}-Domänen, auf. Die variablen Domänen dienen als Bindungsstellen für ein Antigen, wobei eine Bindungsstelle aus einer V_{H}- und einer V_{L}-Domäne ausgebildet ist. Natürliche Antikörper weisen zwei gleiche Bindungsstellen auf, d.h. sie erkennen ein Antigen und werden daher auch als monospezifisch bezeichnet. Künstliche Antikörper können auch zwei verschiedene Bindungsstellen aufweisen, d.h. sie erkennen dann zwei Antigene und werden entsprechend als bispezifisch bezeichnet. Ein Beispiel solcher Antikörper ist jener, der den FcyIIIA Rezeptor (CD16) von natürlichen Killerzellen (NK-Zellen) und das Oberflächenprotein CD30 von Morbus Hodgkin- Zellen erkennt. Mit diesem Antikörper (bimAbHRS-3/A9) können NK-Zellen aktiviert und gegen Morbus Hodgkin-Zellen ausgerichtet werden, wodurch eine Regression von Morbus Hodgkin induziert wird (vgl. DE 43 37 197C sowie Hartmann, F. et al., Blood 89 (1997), 2042). Andererseits hat sich gezeigt, daß bimAbHRS-3/A9 nur schwer herstellbar bzw. reinigungsfähig ist. Darüber hinaus hat sich gezeigt, daß bimAbHRS-3/A9 bei vielen Patienten unerwünschte Immunreaktionen hervorruft.

Ein bispezifisches Fv-Antkörper-Konstruct (Diabody), das nur variable Domänen mit Bindungsstellen für CD19 und CD3 enthält ist in Kipriyanov S. et al. (International Journal of Cancer Bd. 77 (1998), Nr. 5, Seiten 763-772 beschrieben.

Der vorliegenden Erfindung liegt somit die Aufgabe zugrunde, einen Antikörper bereitzustellen, mit dem eine Regression von Morbus Hodgkin induziert werden kann, wobei vorstehende Nachteile vermieden werden.

Erfindungsgemäß wird dies durch die Gegenstände in den Patent-ansprüche erreicht.

Die vorliegende Erfindung beruht auf den Erkenntnissen des Anmelders, daß ein Fᵥ-Antikörper-Konstrukt, das Bindungsstellen für einen CD16-Rezeptor und ein CD30-Oberflächenprotein aufweist, eine Regression von Morbus Hodgkin induzieren kann, wobei die Lyse der Tumorzellen stärker ist als mit bimAbHRS-3/A9. Ferner hat er erkannt, daß ein solches Fᵥ-Antikörper-Konstrukt in großen Mengen und hoher Reinheit hergestellt werden kann. Desweiteren zeichnet sich das Fᵥ-Antikörper-Konstrukt dadurch aus, daß es keine Teile enthält, die zu unerwünschten Immunreaktionen bei Patienten führen können.

Erfindungsgemäß werden die Erkenntnisse des Anmelders genutzt, ein Fᵥ-Antikörper-Konstrukt bereitzustellen, das Bindungsstellen für einen CD16-Rezeptor und ein CD30-Oberflächenprotein aufweist.

Der Ausdruck "Fᵥ-Antikörper-Konstrukt" weist auf ein Antikörper-Konstrukt hin, das variable Domänen, nicht aber konstante Domänen aufweist. Als variable Domänen liegen insbesondere Bindungsstellen für einen CD16-Rezeptor und ein CD30-Oberflächenprotein vor.

Der Ausdruck "Bindungsstelle" weist auf eine V_{H}- und eine V_{L}-Domäne hin, mittels derer das Fᵥ-Antikörper-Konstrukt an einen CD16-Rezeptor bzw. ein CD30-Oberflächenprotein binden kann.

Der Ausdruck "CD16-Rezeptor" umfaßt einen CD16-Rezeptor jeglicher Art und Abstammung. Beispielsweise kann der CD16-Rezeptor von NK-Zellen, Makrophagen oder aktivierten Monocyten stammen. Auch kann der CD16-Rezeptor in Wildtyp- oder veränderter Form vorliegen, wobei letztere Form auch ein Fragment eines CD16-Rezeptors umfaßt, an das ein gegen einen CD16-Rezeptor gerichteter Antikörper binden kann.

Der Ausdruck "CD30-Rezeptor" umfaßt einen CD30-Rezeptor jeglicher Art und Abstammung. Beispielsweise kann der CD30-Rezeptor von Morbus Hodgkin- oder Reed-Sternberg-Zellen stammen. Auch kann der CD30-Rezeptor in Wildtyp- oder veränderter Form vorliegen, wobei letztere Form auch ein Fragment eines CD30-Rezeptors umfaßt, an das ein gegen einen CD30-Rezeptor gerichteter Antikörper binden kann.

Ein erfindungsgemäßes Fᵥ-Antikörper-Konstrukt weist eine oder mehrere Bindungsstellen für einen CD16-Rezeptor und eine oder mehrere Bindungsstellen für ein CD30-Oberflächenprotein auf. Vorzugsweise weist das Fᵥ-Antikörper-Konstrukt eine oder zwei Bindungsstellen für einen CD16-Rezeptor und eine oder zwei Bindungsstellen für ein CD30-Oberflächenprotein auf.

Ein erfindungsgemäßes Fᵥ-Antikörper-Konstrukt kann durch verschiedene Verfahren hergestellt werden. Beispielsweise kann ein Fᵥ-Antikörper-Konstrukt, das eine Bindungsstelle für einen CD16-Rezeptor und eine Bindungsstelle für ein CD30-Oberflächenprotein aufweist, z.B. dadurch hergestellt werden, daß ein erstes einzelkettiges Fᵥ-Antikörper-Konstrukt, das eine V_{H}-Domäne eines anti-CD16-Antikörpers und eine V_{L}-Domäne eines anti-CD30-Antikörpers aufweist, zusammen mit einem zweiten einzelkettigen Fᵥ-Antikörper-Konstrukt, das eine V_{L}-Domäne eines anti-CD16-Antikörpers und eine V_{H}-Domäne eines anti-CD30-Antikörpers aufweist, exprimiert wird, wodurch sich beide aneinanderlagern und das erfindungsgemäße Fᵥ-Antikörper-Konstrukt ausgebildet wird. Ergänzend wird auf die Beispiele 1-3 verwiesen.

Ferner kann ein Fᵥ-Antikörper-Konstrukt, das zwei bis vier Bindungsstellen für einen CD16-Rezeptor und zwei Bindungsstellen für ein CD30-Oberflächenprotein aufweist, z.B. dadurch hergestellt werden, daß ein einzelkettiges Fᵥ-Antikörper-Konstrukt exprimiert wird, das die Elemente (a), und (b) umfaßt:
(a) eine V_{H}-Domäne eines anti-CD16-Antikörpers und eine V_{L} Domäne eines anti-CD30-Antikörpers, wobei die Domänen über einen Peptidlinker 1 miteinander verbunden sind, der jegliche Aminosäuren, insbesondere Glycin (G), Serin (S) und Prolin (P) und vorzugsweise O - 10 Aminosäuren umfassen kann,
(b) eine V_{H}-Domäne eines anti-CD30-Antikörpers und eine V_{L}-Domäne eines anti-CD16-Antikörpers, wobei die Domänen über vorstehenden Peptidlinker 1 miteinander verbunden sind,
wobei die Elemente (a) und (b) über einen Peptidlinker 2 miteinander verbunden sind, der jegliche Aminosäuren, insbesondere Glycin, Serin und Prolin und vorzugsweise 3 - 10 Aminosäuren und ganz besonders die Aminosäuresequenz GGPGS umfassen kann. Ergänzend wird auf die Patentanmeldung 198 19 846.9 des Anmelders verwiesen.

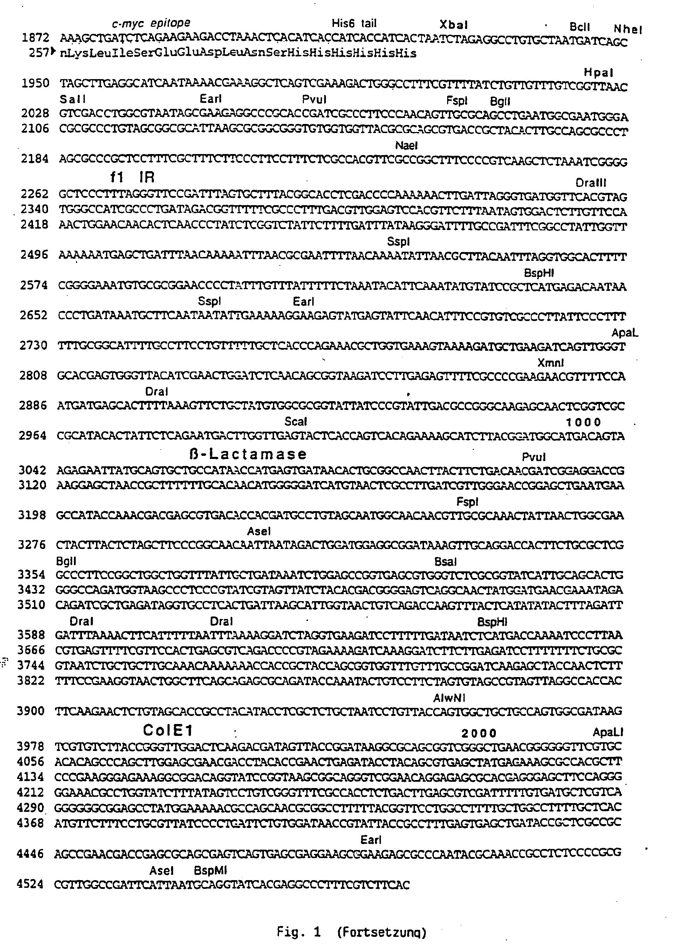

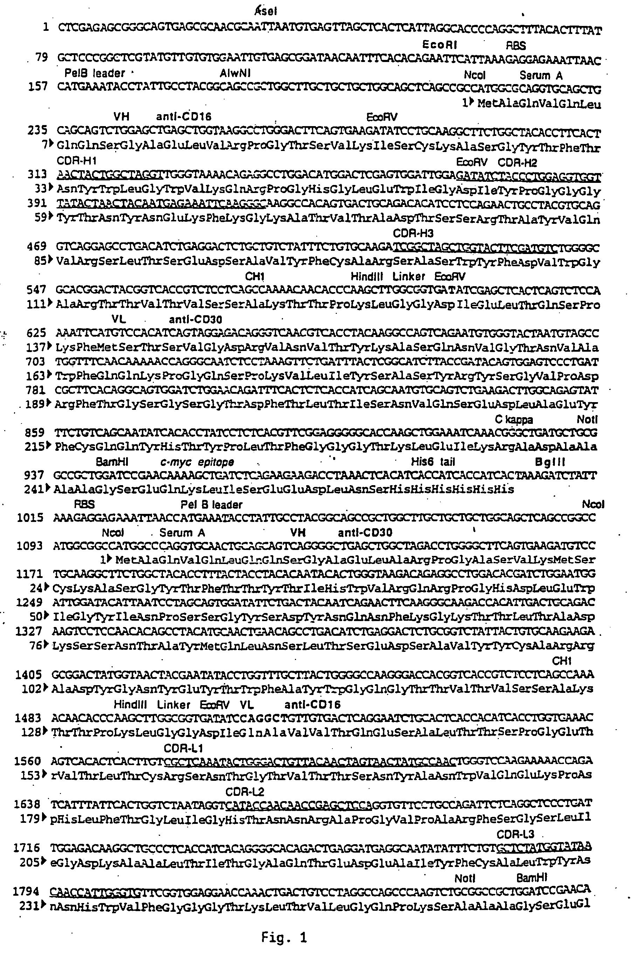

Ein weiterer Gegenstand der vorliegenden Erfindung ist eine Nukleinsäure, insbesondere eine DNA, die für ein vorstehendes Fᵥ-Antikörper-Konstrukt kodiert. Ferner sind Expressions-vektoren, die eine solche DNA enthalten, ein Gegenstand der vorliegenden Erfindung. Bevorzugt wird der Expressionsvektor pKID16-30 von Fig. 1. Dieser wurde bei der DSMZ (Deutsche Sammlung für Mikroorganismen und Zellen) am 29. Juli 1999 unter DSM 12960 hinterlegt. Desweiteren sind Zellen, die einen vorstehenden Expressionsvektor enthalten, ein Gegenstand der vorliegenden Erfindung.

Ein weiterer Gegenstand der vorliegenden Erfindung ist ein Kit, umfassend:
(a) ein erfindungsgemäßes Fᵥ-Antikörper-Konstrukt, und/oder
(b) einen erfindungsgemäßen Expressionsvektor, sowie
(c) übliche Hilfsstoffe, wie Puffer, Lösungsmittel, Träger, Kontrollen und Marker.

Von den einzelnen Komponenten können ein oder mehrere Vertreter vorliegen.

Die vorliegende Erfindung stellt ein Fᵥ-Antikörper-Konstrukt bereit, das Bindungsstellen für einen CD16-Rezeptor und ein CD30-Oberflächenprotein aufweist. Dieses Fᵥ-Antikörper-Konstrukt läßt sich in großen Mengen und großer Reinheit herstellen. Auch weist es keine Teile auf, die zu unerwünschten Immunreaktionen bei Patienten führen können. Besonders kennzeichnet sich das Fᵥ-Antikörper-Konstrukt dadurch, daß es NK-Zellen aktivieren und gegen CD30-Oberflächenproteine exprimierende Zellen, insbesondere Tumorzellen, ganz besonders Morbus Hodgkin- oder Reed-Sternberg Zellen, ausrichten kann, wodurch diese Zellen lysiert werden. Somit eignet sich die vorliegende Erfindung gegen Erkrankungen vorzugehen, bei denen CD30-Oberflächenproteine exprimierende Zellen eine Rolle spielen. Solche Erkrankungen sind z.B. Tumorerkrankungen, insbesondere Morbus Hodgkin.

**Kurze Beschreibung der Zeichnungen:**
- Fig. 1: zeigt den erfindungsgemäßen Expressionsvektor pKID16-30. Dieser kodiert für zwei einzelkettige Fᵥ-Antikörper-Konstrukte, von denen das eine die V_{H}- Domäne eines anti-CD16-Antikörpers und die V_{L}-Domäne eines anti-CD30-Antikörpers und das andere die V_{H}- Domäne eines anti-CD30-Antikörpers und die V_{L}-Domäne eines anti-CD16-Antikörpers aufweist. Nach Expres- sion der einzelkettigen Fᵥ-Antikörper-Konstrukte lagern sich diese aneinander, wodurch ein erfin- dungsgemäßes Fᵥ-Antikörper-Konstrukt erhalten wird.

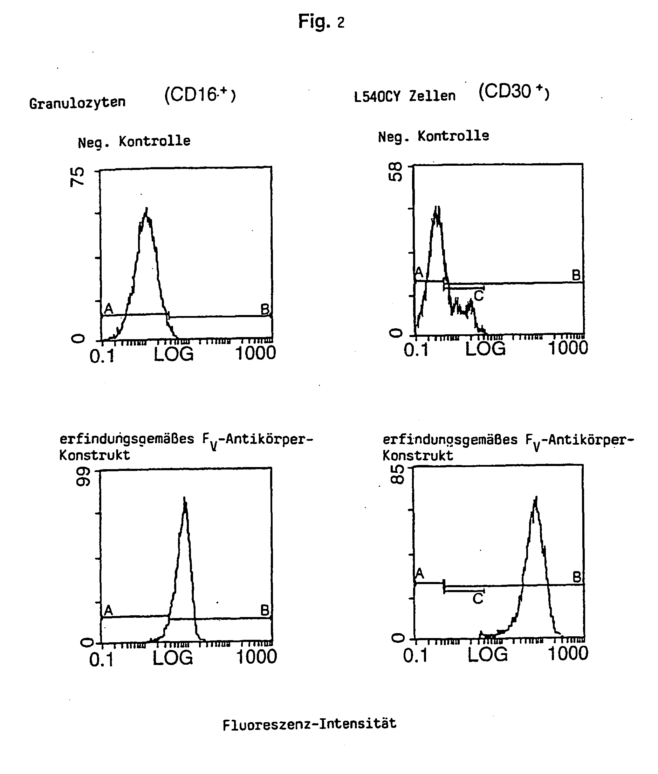

- Fig. 2: zeigt eine FACS-Analyse der Bindung eines erfin- dungsgemäßen Fᵥ-Antikörper-Konstruktes an CD30⁺ L540CY Morbus Hodgkin-Zellen und CD16⁺ Granulocyten. Die Tumorzellen und die Granulocyten wurden jeweils mit 20 µg des erfindungsgemäßen Fᵥ-Antikörper-Kon- struktes inkubiert. Die Bindung des Fᵥ-Antikörper- Konstruktes wurde mit dem anti-c-myc Antikörper 9E10 und Fluorescein-konjugiertem Ziege-anti-Maus IgG bestimmt. Als Negativ-Kontrolle wurden die Zellen alleine mit 9E10 und Fluorescein-konjugiertem Ziege- anti-Maus-IgG inkubiert.

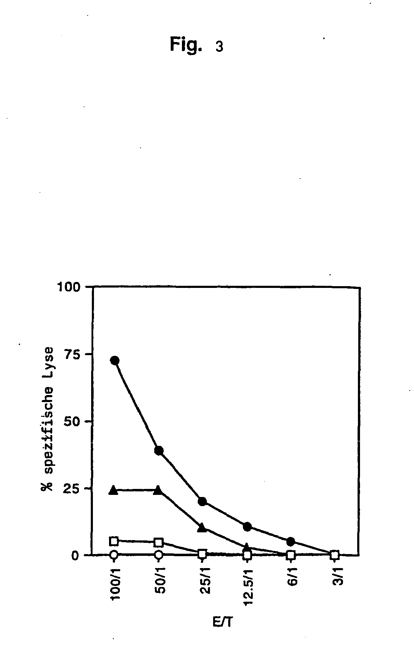

- Fig. 3: zeigt die cytolytische Aktivität von in peripheren Blutlymphozyten (PBL-Zellen) enthaltenen NK-Zellen (Effektor) gegenüber CD30⁺ L540CY Morbus Hogdkin- Zellen (Zielzellen) bei unterschiedlichen Effektor- Zielzellen-Verhältnissesn in einem 5h JAM-Test. Ein erfindungsgemäßes Fᵥ-Antikörper-Konstrukt (●) wurde mit einer Konzentration von 1 µg/ml verabreicht. Als Kontrolle wurde bimAbHRS-3/A9 (▲) (mit einer Konzen- tration von 4 µg/ml verwendet. Als Negativ- Kontrol- le wurden das erfindungsgemäße Fᵥ-Antikörper-Kon- strukt ohne NK-Zellen (o) und NK-Zellen alleine (□) verwendet.

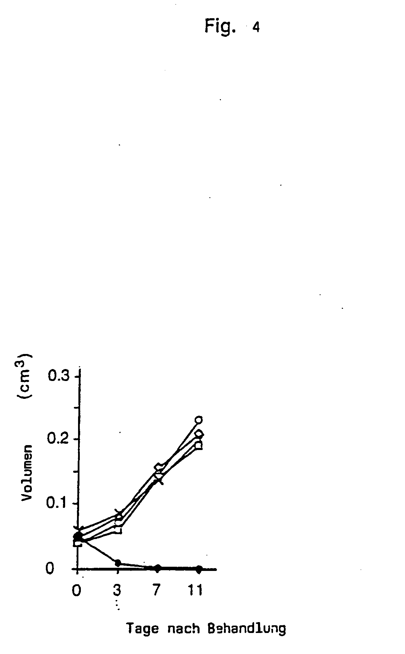

- Fig. 4: zeigt die Behandlung von SCID Mäusen, die Morbus Hodgkin-Xenotransplantate tragen, mit einem erfin- dungsgemäßen Fᵥ-Antikörper-Konstrukt. Die Mäuse wur- den am Tag 0 i.V. mit 100 µg eines erfindungsgemäßen Fᵥ-Antikörper-Konstruktes zusammen mit NK-Zellen enthaltenden PBL-Zellen (●) bzw. ohne solche (o), mit 200 µl PBS (*), mit 1 x 10⁷ PBL-Zellen (□), bzw. mit einem Gemisch von 100 µg mAb HRS-3 und A9 zu- sammen mit PBL-Zellen (◇) behandelt. Tumor-Durch- messer wurden zweimal pro Woche gemessen und das Tumor-Volumen wurde mit folgender Formel berechnet: Volumen = d²x Dxπ/6, wobei d der kleinere und D der größere Tumor-Durchmesser ist.

### Beispiel 1: Konstruktion des erfindungsgemäßen Expressions-vektors pKID16-30

Die cDNA der V_{H}- und V_{L}-Domänen eines anti-CD16-Antikörpers mAb A9 wurde einer PCR unterzogen. Hierfür wurden die folgenden Primer verwendet:
VH5', 5-CAGCCGGCCATGGCGCAGGTC(G)CAGCTGCAGC(G)AG-3 (NcoI);
VH3',5-CCAGGGGCCAGTGGATAGACAAGCTTGGGTGTTGTTTT-3 (HindIII):
VL5',5-AGAGACGCGTACAGGCTGTTGTGACTCAGG-3 (Mlul);
VL3',5-GACTGCGGCCGCAGACTTGGGCTGGCC-3 (NotI) .

Die PCR wurde wie folgt durchgeführt: Ein Zyklus; 5 min bei 94°C, 3 min bei 58°C und 2 min bei 72°C, gefolgt von 30 Zyklen; 80 sec bei 94°C, 80 sec bei 58°C und 2 min bei 72°C bzw. letzteres 10 min im letzten Zyklus. Die PCR-Produkte wurden Gelgereinigt und in den Vektor pCR-Script SK(+) (Stratagene) zur Sequenzierung inseriert. Zur Expression wurde die V_{H}-Domäne über NcoI/HindIII und die V_{L}-Domäne über MluI/NotI in den Vektor pHOG21 inseriert.

Die V_{H}- und V_{L}-Domänen eines anti-CD30-scFᵥ-Fragmentes wurden einer PCR unterzogen. Hierfür wurden die folgenden Primer verwendet:
5-ATGACCATGATTACGCCAAGC-3
5-AGACAAGCTTGGGTTGTTTTGGCTGAGGAGACGG-3 (HindIII);
5-GGCGGATATCGAGCTCACTCAGTCTCC-3 (EcoRV)
5-TATAGCGGCCGCAGCATCAGCCCGTTTGATTTCC-3 (NotI).

Die V_{H}- und V_{L}-Domänen des anti-CD30-scFv-Fragmentes bzw. des anti-CD16-scFv-Fragmentes wurden in den Expressionsvektor pKID inseriert, wodurch der erfindungsgemäße Expressionsvektor pKID 16-30 erhalten wurde. Dieser kodiert für die einzelkettigen Fᵥ-Antikörper-Konstrukte V_{H} 16-V_{L} 30 und V_{H} 30-V_{L} 16.

### Beispiel 2: Expression des erfindungsgemäßen Fᵥ-Antikörper-Konstruktes in Bakterien

E.coli-X11 Blue-Zellen (Stratagene, La Jolla, CA), die mit dem Expressionsplasmid pKID16-30 transformiert worden waren, wurden über Nacht in 2YT-Medium mit 100 µg/ml Ampicillin und 100 mM Glucose bei 37°C gezüchtet. 1:20-Verdünnungen der über Nacht-Kulturen wurden als Kolbenkulturen in 2YT-Medium bei 38°C unter Schütteln mit 280 rpm gezüchtet. Bei einem OD₆₀₀-Wert von 0,8 wurden die Bakterien durch 10 minütige Zentrifugation mit 1500 g bei 20°C pelletiert und in dem gleichen Volumen eines frischen 2YT-Mediums, das 100 µg/ml Ampicillin und 0,4 M Saccharose enthielt, resuspendiert. IPTG wurde mit einer Endkonzentration von 0,1 M zugesetzt und das Wachstum wurde bei 21°C (20-22°C) 18-20 h fortgesetzt. Das Fᵥ-Antikörper-Konstrukt wurde wie in Kipriyanov, S.M. et al., Protein Engineering 10, (1997), 445 beschrieben, isoliert. Anschließend wurde es durch eine Ammoniumsulfatfällung (Endkonzentration 70 % Sättigung) eingeengt. Das Proteinpräzipitat wurde durch Zentrifugation (30000 g, 4°C, 45 min) gewonnen und in 10 % des Anfangsvolumens von 50 mM Tris-HCl, 1 M NaCl, pH 7,0 aufgelöst. Eine immobilisierte Metallaffinitäts-Chromatographie (IMAC) wurde wie in Kipriyanov, S.M. et al., J. Immunol. Methods 200, (1997), 69 beschrieben, durchgeführt. Das gereinigte Fᵥ-Antikörper-Konstrukt wurde gegen eine Phosphat-gepufferte Kochsalzlösung dialysiert.

### Beispiel 3: Charakterisierung des erfindungsgemäßen Fᵥ-Antikörper-Konstruktes

### (A) Durchflußcytometrie

Zum Nachweis der Bindung eines erfindungsgemäßen Fᵥ-Antikörper-Konstruktes an CD16⁺ Granulocyten und CD30⁺ L540CY-Morbus Hodgkin-Zellen wurde eine FACScan (Beckton Dickinson)-Analyse durchgeführt. Hierzu wurden 1 x 10⁶-Zellen zweimal in eiskaltem PBS-N (PBS, 0,05% NaN₃) gewaschen und mit 100 µl des Fᵥ-Antikörper-Konstruktes von Beispiel 2 45 min auf Eis inkubiert. Die Zellen wurden 5 min mit 1200 rpm bei 4°C pelletiert und mit 2 ml PBS-N gewaschen. Die Zellen wurden in 100 µl PBS-N, das 10 µg/ml des an das c-myc bindenden Antikörpers 9E10 (ICI Chemikalien) enthielt, resuspendiert und 30 min auf Eis inkubiert. Die Zellen wurden pelletiert und wie vorstehend gewaschen. Danach wurden die Zellen mit Fluorescein-markiertem Ziege-anti-Maus IgG (Gibco BRL; 1:100 verdünnt in PBS-N), resuspendiert und 30 min auf Eis inkubiert. Nach erneutem Waschen mit PBS-N waren die Zellen für die Analyse mit PBS-N, das 1 µg/ml Propidiumjodid (Sigma) enthielt, bereit. Hintergrund-Fluoreszenz wurde bestimmt, indem die Zellen mit dem Antikörper 9E10 und Fluorescein-markiertem Ziege-anti-Maus-IgG unter gleichen Bedingungen inkubiert wurden.

Es zeigte sich, daß das erfindungsgemäße Fᵥ-Antikörper-Konstrukt sowohl CD16⁺ Granolocyten als auch CD30⁺ L540 CY Morbus Hodgkin-Zellen erkennt und an sie bindet.

### (B) Cytotoxizitätstest

Zum Nachweis der Aktivität eines erfindungsgemäßen Fᵥ-Antikörper-Konstruktes NK-Zellen zu aktivieren, CD30⁺ L540CY Morbus Hodgkin-Zellen zu lysieren wurde ein Cytotoxizitätstest entsprechend des in Matzinger, P., J. Immunol. Meth. 145 (1991), 185 beschriebenen JAM-Tests durchgeführt. In dem Cytotoxizitätstest wird die DNA-Fragmentierung bewertet. Zellen wurden mit [³H] Thymidin bis zu einer Endkonzentration von 2,5-5 µCi/ml für 4-6 h markiert. Die Zellen wurden pelletiert, einmal mit Kulturmedium gewaschen und auf 10⁴ Zellen/Vertiefung einer 96-Lochplatte eingestellt. Nach Zugabe von Effektor-Zellen (NK Zellen enthaltende periphere Blutzellen "PBL-Zellen") in verschiedenen Verdünnungen wurde die 96.Lochplatte in einer befeuchteten Atmosphäre mit 7,5 % CO₂ für 4 h inkubiert. Die Zellen und das Medium wurden auf Fiberglas-Filter gesaugt. Nach Waschen und Trocknen der Filter wurden sie in Plastik-Tüten überfährt, die eine Szintillationsflüssigkeit enthielten und unter Verwendung eines Flüssig-Szintillations-Zählers (LKB) gezählt. Die gemessene Radioaktivität bezieht sich auf intakte DNA, da DNA aus toten Zellen in kleine Fragmente abgebaut ist, die nicht von den Filtern festgehalten werden. Zur Bestimmung der Cytotoxizität, d.h. der Abtötung von Zellen, wurde die Standardformel für den JAM-Test verwendet: % spezifische Abtötung = (S-E)/S 100, wobei E = experimentell erhaltene DNA in Gegenwart von Effektor-Zellen (in cpm) und S = erhaltene DNA in Abweseneheit von Effektor-Zellen (spontan).

Es zeigte sich, daß ein erfindungsgemäßes Fᵥ-Antikörper-Konstrukt NK-Zellen aktivieren kann, CD30⁺ L540CY Morbus Hodgkin-Zellen zu lysieren, wobei die Lyse stärker ist als bei Verwendung von bimAbHRS-3/A9.

### (C) Einfluß auf Tumoren von Mäusen

CD30⁺ L540CY Hodgkin's Lymphome wurden in SCID Mäusen, wie in Hombach, A. et al., Int. J. Cancer 55, (1993), 830; Renner, C. et al., J. Hematotherapy 4, (1995), 447 beschrieben, etabliert. Hierzu wurden 1.5x10⁷ Tumorzellen in 200µl PBS subcutan in die rechte Flanke der Mäuse injiziert. Die Tumor-Entwicklung, d.h. der Tumor-Durchmesser, wurde zweimal pro Woche bestimmt. Mäuse mit Tumoren von 4-6 mm im Durchmesser wurden in verschiedene Gruppen eingeteilt und erhielten ein erfindungsgemäßes Fᵥ-Antikörper-Konstrukt in 200µl PBS zusammen mit NK-Zellen enthaltenden peripheren Blutlymphozyten (PBL-Zellen). Das Tumorvolumen und seine Entwicklung wurden bestimmt (vgl. Legende zu Fig. 4).

Es zeigte sich, daß ein erfindungsgemäßes Fᵥ-Antikörper-Konstrukt nicht nur in vitro sondern auch in vivo NK-Zellen aktivieren kann, CD30⁺ L540CY Morbus Hodgkin-Zellen zu lysieren.

## Patentansprüche

1. Fv-Antikörper-Konstrukt, das variable Domänen, nicht aber konstante Domänen aufweist, wobei die variablen Domänen Bindungsstellen für einen CD16-Rezeptor und ein CD30-Oberflächenprotein umfassen.

2. Fv-Antikörper-Konstrukt nach Anspruch 1, wobei der CD16-Rezeptor von NK-Zellen stammt.

3. Fᵥ-Antikörper-Konstrukt nach Anspruch 1 oder 2, wobei das CD30-Oberflächenprotein von Morbus Hodgkin- oder Reed-Sternberg-Zellen stammt.

4. Fᵥ-Antikörper-Konstrukt nach einem der Ansprüche 1 - 3, wobei jeweils eine Bindungsstelle vorliegt.

5. Fᵥ-Antikörper-Konstrukt nach Anspruch 4, kodiert durch den Expressionsvektor pKID16-30 (DSM 12960).

6. Fᵥ-Antikörper-Konstrukt nach einem der Ansprüche 1-3, wobei jeweils zwei Bindungsstellen vorliegen.

7. Expressionsvektor, kodierend für das Fᵥ-Antikörper-Konstrukt nach einem der Ansprüche 1 - 6.

8. Expressionsvektor nach Anspruch 7, nämlich pKID16-30 (DSM 12960).

9. Transformante, enthaltend den Expressionsvektor nach Anspruch 7 oder 8.

10. Verfahren zur Herstellung des Fᵥ-Antikörper-Konstruktes nach einem der Ansprüche 1-6, umfassend die Kultivierung der Transformante nach Anspruch 9 unter geeigneten Bedingungen.

11. Kit, umfassend:
(a) ein Fᵥ-Antikörper-Konstrukt nach einem der Ansprüche 1-6, und/oder
(b) einen Expressionsvektor nach Anspruch 7 oder 8, sowie
(c) übliche Hilfsstoffe, wie Puffer, Lösungsmittel, Träger, Kontrollen und Marker,
wobei von den einzelnen Komponenten ein oder mehrere Vertreter vorliegen können.

12. Fᵥ-Antikörper-Konstrukt nach einem der Ansprüche 1 bis 6 zur Verwendung als Arzneimittel..

13. Verwendung des Fᵥ-Antikörper-Konstruktes nach einem der Ansprüche 1 bis 6 zur Herstellung eines Medikaments zur Lyse von CD30-Oberflächenproteinen exprimierenden Zellen .

14. Verwendung nach Anspruch 13, wobei das Medikament zur Behandlung von Tumorerkrankungen, insbesondere von Morbus Hodgkin- oder Reed-Tumoren ist.

## Claims

1. Fᵥ antibody construct, comprising variable domains but not constant domains, wherein the variable domains contain binding sites for an CD16 receptor and a CD30 surface protein.

2. Fᵥ antibody construct according to claim 1, wherein the CD16 receptor is derived from NK cells.

3. Fᵥ antibody construct according to claim 1 or 2, wherein the CD30 surface protein is derived from Hodgkin's disease or Reed-Sternberg cells.

4. Fᵥ antibody construct according to any of claims 1 to 3, wherein one binding site is present each.

5. Fᵥ antibody construct according to claim 4, encoded by the expression vector pKID16-30 (DSM 12960).

6. Fᵥ antibody construct according to any of claims 1 to 3, wherein two binding sites are present each.

7. Expression vector, coding for the Fᵥ antibody construct according to any of claims 1 to 6.

8. Expression vector according to claim 7, namely pKID16-30 (DSM 12960).

9. Transformant, containing the expression vector according to claim 7 or 8.

10. A method of producing the Fᵥ antibody construct according to any of claims 1 to 6, comprising culturing the transformant according to claim 9 under suitable conditions.

11. Kit comprising:
(a) an Fᵥ antibody construct according to any of claims 1 to 6, and/or
(b) an expression vector according to claim 7 or 8, and
(c) common auxiliary substances, such as buffers, solvents, carriers, controls and markers,
wherein one or more representatives of the individual components may be present.

12. Fᵥ antibody construct according to any of claims 1 to 6 for use as a drug.

13. Use of an Fᵥ antibody construct according to any of claims 1 to 6 for the production of a drug for lysis of cells expressing CD30 surface proteins.

14. Use according to claim 13, wherein the drug is for treatment of tumor diseases, especially of Hodgkin's disease or Reed-Sternberg cells.

## Revendications

1. Construction d'anticorps Fᵥ comportant des domaines variables, toutefois pas de domaines constants, les domaines variables comprenant des sites de liaison pour un récepteur CD16 et une protéine de surface CD30.

2. Construction d'anticorps Fᵥ selon la revendication 1, le récepteur CD16 étant issu de cellules NK.

3. Construction d'anticorps Fᵥ selon la revendication 1 ou 2, la protéine de surface CD30 étant issue de cellules Morbus de Hodgkin ou de Reed-Sternberg.

4. Construction d'anticorps Fᵥ selon l'une des revendications 1 à 3, dans laquelle est chaque fois présent un site de liaison.

5. Construction d'anticorps Fᵥ selon la revendication 4, que code le vecteur d'expression pKID16-30 (DSM 12960).

6. Construction d'anticorps Fᵥ selon l'une des revendications 1 à 3, dans laquelle sont chaque fois présents deux sites de liaison.

7. Vecteur d'expression codant la construction d'anticorps Fᵥ selon l'une des revendications 1 à 6.

8. Vecteur d'expression selon la revendication 7, notamment pKID16-30 (DSM 12960).

9. Transformants, contenant le vecteur d'expression selon la revendication 7 ou 8.

10. Procédé de préparation de la construction d'anticorps Fᵥ selon l'une des revendications 1 à 6, comprenant la culture des transformants selon la revendication 9 dans des conditions appropriées.

11. Kit, comprenant :
(a) une construction d'anticorps Fᵥ selon l'une des revendications 1 à 6, et/ou
(b) un vecteur d'expression selon la revendication 7 ou 8, ainsi que
(c) des substances auxiliaires habituelles, telles que tampon, solvants, porteurs, contrôles et marqueurs,
des différents composants pouvant être présents un ou plusieurs représentants.

12. Construction d'anticorps Fᵥ selon l'une des revendications 1 à 6 destinée à être utilisée comme médicament.

13. Utilisation de la construction d'anticorps Fᵥ selon l'une des revendications 1 à 6 pour la préparation d'un médicament pour lyser des cellules exprimant des protéines de surface CD30.

14. Utilisation selon la revendication 13, le médicament servant pour le traitement de tumeurs, en particulier de tumeurs de Morbus de Hodgkin ou de Reed.
